# EUROPEAN PATENT APPLICATION

(11) **EP 4 218 656 A1**
(43) Date of publication of application: **02.08.2023**
(21) Application number: 21873041.4
(22) Date of filing: 10.09.2021
(51) Int. Cl.: A61B 90/11

(54) **METHOD FOR POSITIONING A MEDICAL INSTRUMENT FOR INVASIVE SURGERY**

(30) Priority: 22.09.2020 RU 2020131294
(71) Applicant: Obshchestvo s Ogranichennoi Otvetstvennostiu «Interventsionnye Radiologicheskie Sistemy», Moscow, 121205 (RU)
(72) Inventor: EVTEEV, Viacheslav Valerevich, Voronezh, 394086 (RU); IAGODKINA, Irina Aleksandrovna, Voronezh, 394077 (RU); GREBENNIKOV, Aleksei Petrovich, Voronezh, 394036 (RU); CHERNYH, Mikhail Anatolyevich, Voronezh, 394005 (RU)
(74) Representative: Perronace, Andrea
(86) International application number: PCT/RU2021/050292
(87) International publication number: WO 2022/066054

(57) **Abstract**

The group of inventions relates to medicine, namely, X-ray computed tomography (X-ray CT) guided surgical procedures and means of surgical instrument positioning and orienting, e.g. in diagnostic radiology. The technical result is the improvement of medical instrument positioning quality under various human body movements during surgery by visual and radiological CT monitoring of dislocation of tissues, organs and human body parts during surgery, and the possibility of precise measurements of dislocation and timely correction of respiratory excursions during surgery. A pre-surgery diagnostic examination is performed, an operation area is determined. An RLM oriented in a direction corresponding to a coronal plane is placed under a patient's body on a diagnostic equipment table surface. An RCG is located over a patient's body surface in a planned surgery area in a way that internal jumpers are oriented in a sagittal plane while outer protrusions are oriented in a coronal plane. The patient is instructed to breathe in and out several times and the dislocation of outer protrusions of the RCG relative to the line made by the laser illumination of the diagnostic equipment and relative to the RLM located under the patient's body on the table is evaluated. The patient is trained to breathe with a required amplitude. The instrument insertion site in a human body is marked with a sterile marker. A stereotactic holder is placed in an instrument insertion site. Position (dislocation) of MR-opaque protrusions of a stereotactic holder base and RCG external protrusions is checked.

## Description

The group of inventions is related to medicine, namely, X-ray computed tomography (X-ray CT)-guided surgical procedures and means of surgical instrument positioning and orienting, e.g. in diagnostic radiology.

The problem is in change of a spatial position of a medical instrument introduced into a human body due to human body movements, organs and tissues disposition during a surgical procedure (caused by various amplitude respiratory excursions, skeletal muscles contractions, internal organ disposition due to peristalsis). Changes in spatial position of instruments lead to a decreased precision of a procedure, require additional alignments control, increase the radiation exposure and surgery time and risk of complications, deteriorate the quality of surgical procedures and require additional equipment for monitoring.

This problem is solved in several different ways: by using technical real-time hardware movement monitoring; by using stereotactic devices; by using repeated diagnostic series and stepwise insertion of medical instruments into the human body; by using robots to perform CT-guided surgical procedures. However, there is no universal and effective solution to the above problem as for the state of the art of medicine and technology.

Thus, state-of-the-art conventional real-time monitoring solutions employed by CT scanner manufacturers do not allow using scanning block sizes required for a full control of instrument movement due to various angles, amplitudes and complexity of movements. Scanning blocks of modern tomographs may include a few to tens of slices with a block length in the range of 3 mm to 20-30 mm. Meanwhile, the movement of certain parts of the human body due to respiratory excursions may exceed up to 5-6 cm and real-time monitoring is not suitable for complete accommodation and control of such spatial changes. It should be noted that the use of a real-time monitoring mode is limited in time due to additional radiation exposure of the patient and medical staff; the duration offered by some CT scanner vendors is only 60 to 90 seconds, what may be insufficient for a CT-guided surgical procedure.

Real-time X-ray monitoring solutions that are not conventional for a CT scanner (X-ray television fluoroscopy equipment) are characterized by a lower scanning resolution and inferior visualization, so the procedure is not as detailed as in CT scanning modality. This approach requires an integration of additional output devices to user interfaces, it requires rapid human-assisted analysis and interpretation (within a few seconds) in order to make decision and subsequent correct positioning of the instrument or human body. Such integration currently still requires development of medical image processing algorithms, improvements of instrumental and hardware components of utilized medical equipment, high computing capacity of workstations, using artificial intelligence to aid a human in fast integration, analysis, and interpretation of data generated during a surgical procedure, which remains an unsolved problem of the state-of-the-art medicine and technology. It should be noted that so-called real-time monitoring is provisional and only means a monitoring with a higher scanning frequency and higher frame rate of obtained images.

Real-time monitoring devices based on other physical principles (alternative to X-ray radiation, such as ultrasound units or electromagnetic or optical control systems) have the same disadvantages as X-ray units. Its application is limited by more heterogeneous data transmitted to a user interface. The state-of-the-art technology does not provide fast and direct interaction during analysis of data transmitted by such means due to differences in vendor approach, property rights, interface protocols, etc. Some control units do not provide information on a lesion and internal organs, just reflecting spatial interrelations between its sensors (electromagnetic monitoring means) or an instrument position on a human body surface (optic navigation systems).

The available devices of passive monitoring, including stereotactic devices, act as a markup to maintain a medical instrument in a required spatial position, but do not provide the ability of respiratory movement or spontaneous movemens assessment.

There is a well-known group of patient-individualized surgical instrument positioning and orientation in pathway formation devices, it provide a method of invasive surgery instruments positioning. This process includes steps of detecting a pathway position for a medical instrument, obtaining individual patient's data and choosing an optimal position for placing an orienting device implemented as a stereotactic medical instrument holder. It is presented by a trajectory indicator installed in arch-shaped brackets fixed in a ring-shaped base and rotatable in two mutually perpendicular vertical planes components (RU 2 662 872 C2 , MΠK A61B 34/20 (2016.01), A61B 90/11 (2016.01)).

A radiopaque grid for surgical field markup is well validated device. It contains of a basal layer made of a radiolucent material and radiopaque filaments incorporated in a rectangular grid with rectangular cells dimensioned of 20 to 50 mm. One side of a base has an adhesive layer along the perimeter for fixation to patient's skin (RU 152847 U1, publ. June 20, 2015, Bul. No. 17).

A stereotactic medical instrument holder including a trajectory indicator installed in arch-shaped brackets fixed in a ring-shaped base and rotatable in two mutually perpendicular vertical planes is also developed and validated (description for Patent RU 2569720, MΠK A61B 19/00 (2006.01), published on November 27, 2015).

The objective of the invention is to develop a mean for monitoring and navigation for performing CT-guided surgical procedures in medicine, radiology, surgery and medical technology.

The technical result is an improvement of medical instrument positioning quality under conditions of various human body movements during surgery by means of visual and radiological CT monitoring of tissue, organ and human body parts dislocation during surgery, ability of precise measurements of dislocation and in-time repositioning correction due to respiratory excursions during surgery.

The technical result is achieved by a method of invasive surgery instrument positioning containing steps where the instrument pathway position is determined, individual patient's data is collected and an optimal position is determined for locating an orienting device implemented as a stereotactic medical instrument holder including a trajectory indicator installed in arch-shaped brackets fixed in a ring-shaped base and rotatable in two mutually perpendicular vertical planes; by a data collection step including determination of a patient's body surface dislocation during respiration and a corresponding respiratory movements amplitude by locating a radiopaque linear marker on a surgery table under a patient's body in the axial plane of the surgery object and by locating a coordinate radiopaque gride implemented as a rectangular frame with transverse jumper forming internal rectangular cells and two rows of outer rectangular cells located along transverse sides of a frame over a patient's body in a planned surgery area. The grid is located in such a way that internal cell jumpers are oriented in a sagittal plane and outer cell jumpers are oriented in an axial plane, and the magnitude of a patient's body dislocation during respiration is assessed by a dislocation of outer cell jumpers in relation to a linear marker on a surgical table and in relation to a line created by laser illumination of a diagnostic equipment. If a body region movement amplitude is 15-30 mm or less, lines are drawn on the skin in axial and sagittal planes, marking a medical instrument insertion site with subsequent installing in the latter a stereotactic holder having four pairs of radiopaque markers shaped as parallel protrusions located on a base on both sides of brackets fixtures, while a holder is installed in such a way that an axial and a sagittal marker lines are located between protrusions.

A coordinate radiopaque grid (RCG) for a surgical field markup is presented by a rectangular frame with transverse jumper forming internal rectangular cells and has a number of outer rectangular cells on each transverse side of a frame.

A stereotactic medical instrument holder including a trajectory indicator installed in arch-shaped brackets fixed in a ring-shaped base and rotatable in two mutually perpendicular vertical planes is additionally equipped with four pairs of radiopaque markers shaped as parallel protrusions located on a base on both sides of brackets fixtures.

It is schematically described in Fig.1, Fig.2, Fig.3, Fig.4, Fig.5 the steps of selection of an invasive surgery instrument position; Fig.6 illustrates a coordinate radiopaque grid (RCG); Fig. 7 illustrates a stereotactic holder; Fig.8 illustrates a stereotactic holder radiopaque markers pair.

RCG markup instrument for surgical field markup (Fig.6) is a rectangular-shaped frame with transverse parallel jumpers forming internal rectangular cells. Each transverse side of a frame has a number of outer rectangular cells with sides perpendicular to transverse jumper, forming outer protrusions.

The resilience and rigidity of RCG construction elements may vary.

The distance between outer protrusions and internal jumper may be different. The distance between protrusions on contralateral sides of a frame may be various.

RCG may have graduation with different graduation values.

Stereotactic holder (Fig.7) has four pairs of radiopaque markers shaped as parallel protrusions (Fig.8) located at an area of upper and lower segments' fixture to a base. The protrusions are developed for alignment with corresponding RCG and RLM construction elements. The protrusions may be located along the outer and/or inner circumference of a stereotactic holder base and may have different opacities under fluoroscopy, X-ray computed tomography or magnetic resonance imaging.

A stereotactic holder may be equipped with fixation outfit to adhere an underlying surface (adhesive base, dressing, adhesive tape, suture holes, etc.)

RLM markup instrument may have graduation in different graduation values.

The method of invasive surgery instrument positioning includes the following steps.

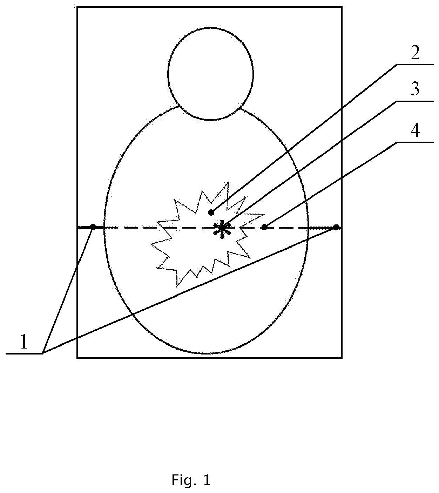

1st. A radiopaque linear marker (RLM) oriented in axial plane is placed under a patient's body on a diagnostic equipment table in a plane of a planned intervention. A RLM markup instrument position corresponding to an axial plane is controlled by a CT scanner midline laser in axial plane (Fig. 1). A RLM markup instrument may have graduation.

2nd. A pre-surgery diagnostic examination is performed:
- a surgical procedure area is determined,
- a surgery object is detected,
- a required slice number locating a surgery object and a medical instrument insertion site on a human body surface is determined.

3rd. A slice number locating a closest RLM markup instrument (relative to the surgery object slice number and medical instrument insertion site on the human body surface as determined at the previous step) is determined. If a single RLM markup was placed, the slice corresponding to it is chosen.

4th. A CT scanner table with a patient on it is set up in a position where RLM is aligned with the CT scanner midline laser (Fig. 1.).

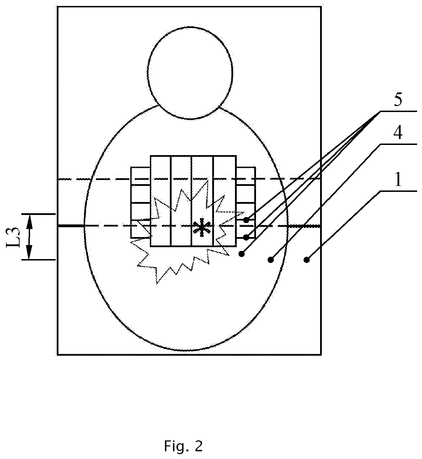

5th. A RCG is located over a patient's body surface in a planned surgery area in a way that internal transverse jumpers are oriented in a sagittal plane while outer protrusions are oriented in an axial plane. Here, a slice of chosen (or a single) RLM markup instrument with the axial line of a midline laser should be located in a slice array that includes RCG. RCG is located over RLM (Fig. 2).

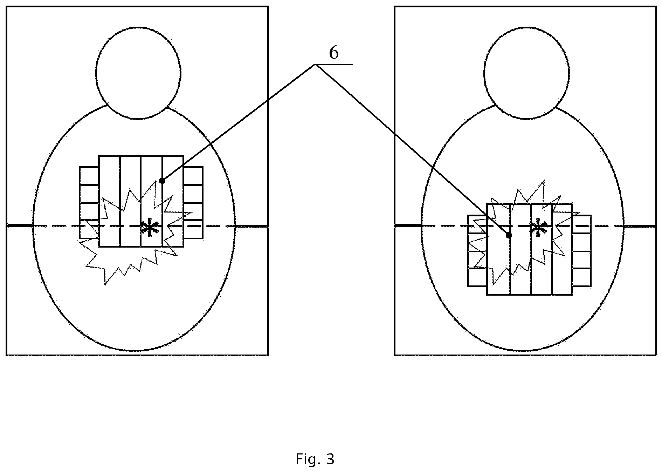

6th. The patient is instructed to breathe in and out several times and the dislocation of outer protrusions of the RCG markup instrument relative to the line made by the laser illumination of the diagnostic equipment and relative to the RLM markup instrument located under the patient's body on the diagnostic equipment table is evaluated. The dislocation is registered by visual inspection. A dislocation may be estimated approximately by counting RCG markup instrument outer protrusions located above or below relative to RLM markup instrument and a midline lacer axial line aligned with each other. A dislocation may be assessed more precisely in mm using corresponding measuring instruments graded in mm (Fig. 2, Fig. 3, Fig. 6 and Fig. 7).

Implementation of the 6th step provides information on the dislocation amplitude of a human body fitted with a grid markup tool in relation to stable markers (RLM markup tool aligned with a midline laser axial line). A dislocation value most comfortable for a patient and a corresponding respiratory movements amplitude are chosen. The patient is instructed on a requirement to breathe with a certain depth. The patient is trained to breathe with a required depth by performing several breathing movement until a required result is achieved as a dislocation with an amplitude chosen.

7th. Intraoperative CT examination is performed with a scope limited to the surgery area mandatory including RLM and considering their dislocation amplitude. The value of a patient's body surface dislocation due to breathing is assessed by dislocation of outer cell jumpers relative to RLM linear marker on a table and relative to a line created by a diagnostic equipment laser illumination; if the body surface dislocation is 15-30 mm or less, lines are drawn on the skin in axial and sagittal planes, marking a medical instrument incretion site with subsequent installing in the latter a stereotactic holder,

7th. The data obtained on a 7th step are subject to a multiplanar reconstruction using a CT scanner software. An axial slice containing an instrument insertion site is chosen. A position of this slice on a patient's skin may be obtained by moving a table to a required slice number with a subsequent illumination by a midline laser axial line or by calculating a distance in millimeters from a RCG markup tool upper or lower edge. A markup line in axial plane passing through a chosen axial slice is made on the skin with a marker. The sagittal slice containing the instrument insertion site is chosen. The position of this slice on the skin may be obtained by calculating the closest internal jumper of a RCG markup instrument or by calculating a distance in millimeters from a RCG markup instrument left or right side. A markup line in sagittal plane passing through a chosen sagittal slice is made on the skin with a marker. Thus, an instrument insertion site on a patient's skin is located at an intersection of the two above lines: axial and sagittal.

At the 9th step, a RCG may be removed from the patient's body surface; if indicated, it may be left in the position described in step 5.

10th. A stereotactic holder is placed in the marked instrument insertion site in a way that stereotactic holder base protrusions are aligned with the axial and sagittal markup lines.

11th. A stereotactic holder is fixed to the patient's skin and is adjusted to a required tilt angle.

12th. The dislocation of the stereotactic holder base protrusions in axial plane is checked as in step 6 by visually inspecting a dislocation relative to a RLM aligned with a midline laser axial line.

13th. The next intraoperative CT examination of a surgical area is performed with detection of surgical site organ, RLM markup instrument and stereotactic holder base protrusions locations are determined. Correspondence to a chosen trajectory is assessed. If necessary, a stereotactic holder tilt angle is adjusted and patient's respiratory movements amplitude are adjusted.

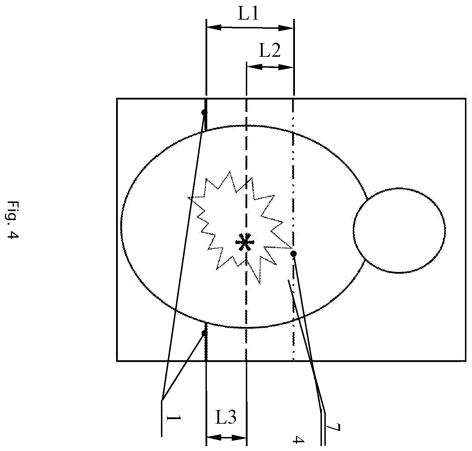

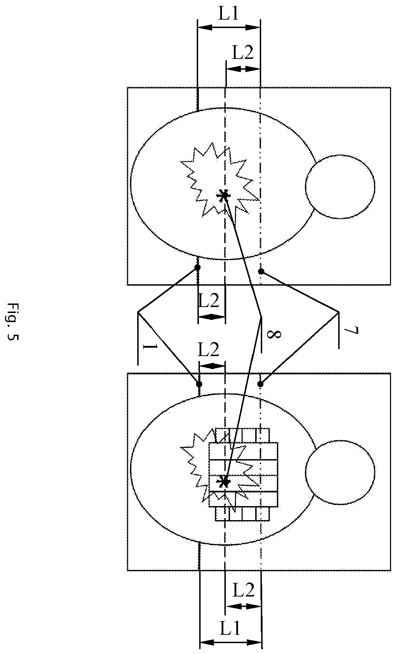

14th. The medical instrument is inserted into the human body with insertion controlled by stepwise measures at step 13 or as a single-step with additional control by real-time monitoring. Stereotactic holder protrusions dislocation and respiratory movements amplitude during insertion are controlled as in step 13. If a CT scanner table with a patient was moved in any direction (e.g. to create more space and freedom of movement for an operator) and a plane of midline laser axial line is not aligned with a plane of a RLM markup instrument, a dislocation of stereotactic holder base protrusions is assessed visually in relation to a current position of a midline laser axial line (Fig. 4 and Fig. 5).

15th. Surgical access to a desired site.

16th. A follow-up CT examination is performed, after which a medical instrument and stereotactic holder are withdrawn and a surgery is completed.

## Claims

1. A method of invasive surgery instrument positioning containing steps where a medical instrument pathway position is determined, individual patient's data are collected and an optimal position is determined for locating an orienting device implemented as a stereotactic medical instrument holder including a trajectory indicator installed in arch-shaped brackets fixed in a ring-shaped base and rotatable in two mutually perpendicular vertical planes, **characterized in that** a data collection step including determination of a patient's body surface dislocation during respiration and a corresponding respiratory movement amplitude by locating a radiopaque linear marker on a surgery table under a patient's body in axial plane of a surgery object and by locating a coordinate radiopaque grid implemented as a rectangular frame with transverse jumper forming internal rectangular cells and two rows of outer rectangular cells located along transverse sides of a frame over the patient's body in a planned surgery area, the grid is located in such a way that internal cell jumpers are oriented in a sagittal plane and outer cell jumpers are oriented in an axial plane, where the magnitude of a patient's body dislocation during respiration is assessed by a dislocation of outer cell jumpers in relation to a linear marker on a surgical table and in relation to a line created by laser illumination of the diagnostic equipment; if the body surface movement is 15-30 mm or less, lines are drawn on the skin in axial and sagittal planes, marking the medical instrument insertion site with subsequent installation in the latter of a stereotactic holder having four pairs of radiopaque markers shaped as parallel protrusions located on a base on both sides of brackets fixtures, while a holder is installed in such a way that the axial and sagittal marker lines are located between protrusions.

2. A coordinate radiopaque grid for a surgical field markup as a rectangular frame with transverse jumper forming internal rectangular cells, **characterized in that** it has a number of outer rectangular cells on each transverse side of a frame.

3. A stereotactic medical instrument holder including a trajectory indicator installed in arch-shaped brackets fixed in a ring-shaped base and rotatable in two mutually perpendicular vertical planes, **characterized in that** it is equipped with four pairs of radiopaque markers shaped as parallel protrusions located on a base on both sides of brackets fixtures.
